(19) Europäisches Patentamt
European Patent Office
Office européen des brevets

(11) Veröffentlichungsnummer: **0 097 297**
**B1**

# (12) EUROPÄISCHE PATENTSCHRIFT

(45) Veröffentlichungstag der Patentschrift: **27.12.85**

(51) Int. Cl.⁴: **C 07 D 239/56**, C 07 D 239/60, A 61 K 31/505

(21) Anmeldenummer: **83105762.5**

(22) Anmeldetag: **13.06.83**

(54) **Substituierte 5-Phenylthio-6-amino-pyrimidinone, Verfahren zu ihrer Herstellung und ihre Verwendung sowie diese Verbindungen enthaltende Zubereitungen.**

(30) Priorität: **18.06.82 DE 3222914**

(43) Veröffentlichungstag der Anmeldung: **04.01.84 Patentblatt 84/1**

(45) Bekanntmachung des Hinweises auf die Patenterteilung: **27.12.85 Patentblatt 85/52**

(84) Benannte Vertragsstaaten: **AT BE CH DE FR GB IT LI LU NL SE**

(56) Entgegenhaltungen: **GB - A - 990 857**

(73) Patentinhaber: **Beiersdorf Aktiengesellschaft, Unnastrasse 48, D-2000 Hamburg 20 (DE)**

(72) Erfinder: **Cohnen, Erich, Dr., Moorende 74, D-2155 Jork (DE)**
Erfinder: **Armah, Ben, Dr., Milcher Strasse 9 d, D-2000 Hamburg 52 (DE)**

EP 0 097 297 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Gegenstand der Erfindung sind substituierte 5-Phenylthio-6-amino-pyrimidinone der allgemeinen Formel I

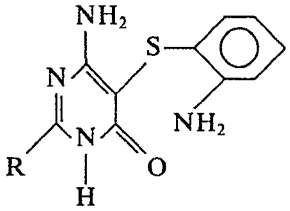

(I)

in der R eine Amino- oder Hydroxygruppe bedeutet, sowie ihre tautomeren Formen und deren Säureadditionssalze, Verfahren zu ihrer Herstellung und ihre Verwendung in pharmazeutischen Präparaten.

Der Einfachheit halber sind die erfindungsgemäßen Verbindungen in nur einer durch Formel I wiedergegebenen tautomeren Form definiert. Die Erfindung erstreckt sich jedoch auf alle tautomeren Formen der Verbindungen, insbesondere auf das Pyrimidindion.

Obgleich pharmazeutisch verträgliche Salze der neuen Verbindungen der Formel I und deren tautomere Formen bevorzugt sind, liegen alle Säureadditionssalze innerhalb des Bereichs der Erfindung. Alle Säureadditionssalze sind wertvoll zur Herstellung der Basen, selbst wenn das spezielle Salz nur als Zwischenprodukt gewünscht wird, wie z. B., wenn das Salz nur für Zwecke der Reinigung oder Identifizierung gebildet wird, oder wenn es als ein Zwischenprodukt bei der Herstellung eines pharmazeutisch verträglichen Salzes, z. B. durch Ionenaustauschverfahrensweisen, verwendet wird.

Die folgenden Verbindungen der allgemeinen Formel I und deren Salze werden besonders bevorzugt:

6-Amino-5-(2-amino-phenylthio)-2,4(1 H, 3 H)-pyrimidindion. Diese Verbindung und ihre tautomeren Formen sind neu.

2,6-Diamino-5-(2-amino-phenylthio)-4(1 H)-pyrimidinon. Diese Verbindung und ihre tautomeren Formen sind neu, ausgenommen 2,4-Diamino-5-(2-aminophenylthio)-6-hydroxypyrimidin.

Aus der britischen Patentschrift 990 857 ist die Verbindung 2,4-Diamino-5-(2-aminophenylthio)-6-hydroxypyrimidin als Zwischenprodukt bekannt. Ein therapeutischer Verwendungszweck ist nicht angegeben.

Die erfindungsgemäßen Verbindungen besitzen ausgeprägte diuretische, saluretische und uricosurische Wirkungen und können daher zur Behandlung von Hypertonie, Hyperuricämie und Ödemen verwendet werden.

Die Verbindungen der Erfindung können beim Menschen oral oder parenteral, vorzugsweise in einer Dosierung von 250—1 000 mg pro Tag, angewendet werden, insbesondere in unterteilten Dosen, zum Beispiel zweimal täglich.

Zur Hypertoniebehandlung werden bevorzugt Dosen von 500—1 000 mg, verteilt auf zwei Gaben, täglich verabreicht. Diese Dosierung gilt auch für die Behandlung von Ödemen. Die Behandlung der Hyperuricämie erfolgt vorzugsweise mit 250 mg bis 500 mg pro Tag.

Gemäß der Erfindung werden auch pharmazeutische Zusammensetzungen geschaffen, die eine Verbindung der allgemeinen Formel I, in der R eine Amino- oder Hydroxygruppe bedeutet, oder deren pharmazeutisch verträgliche Salze, zusammen mit einem pharmazeutisch verträglichen Verdünnungsmittel oder Träger enthalten.

Die Verbindungen gemäß der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können oral in Form von Tabletten, Dragees, Sirups, Suspensionen und Flüssigkeiten, oder parenteral in Form von Lösungen oder Suspensionen verabreicht werden. Oral zu verabreichende Präparate können ein oder mehrere Zusätze, wie Süßungsmittel, Aromatisierungsmittel, Farbstoffe und Konservierungsmittel, enthalten. Tabletten können den Wirkstoff mit üblichen pharmazeutisch verträglichen Hilfsmitteln vermischt enthalten, zum Beispiel inerten Verdünnungsmitteln wie Calciumcarbonat, Natriumcarbonat, Lactose und Talk, Granulierungsmitteln und Mitteln, die den Zerfall der Tabletten bei oraler Verabreichung fördern, wie Stärke oder Alginsäure, Bindemitteln wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Äthanol, Propylenglycol, Äther des Tetrahydrofurylalkohols und Wasser.

Die Tabletten können nach bekannten Arbeitsweisen überzogen werden, um den Zerfall und die Resorption im Magen-Darmtrakt zu verzögern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, zum Beispiel Suspendiermitteln wie Methylcellulose, Tragacanth oder Natriumalginat, Netzmitteln wie Lecithin, Polyäthylenstearat und Polyoxyäthylensorbitanmonooleat, und Konservierungsmitteln wie Äthylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert. Die pharmazeutischen Präparate können den Wirkstoff in einer Menge von 0,1 bis 90%, insbesondere 1 bis 90%, enthalten, wobei der Rest ein Trägerstoff oder Zusatzstoff ist. Im Hinblick auf die Herstellung und Verabreichung werden feste Präparate, wie Tabletten und Kapseln, bevorzugt. Vorzugsweise enthalten die Präparate den Wirkstoff in einer Menge von 250 mg.

Die Herstellung der erfindungsgemäßen Pyrimidinone der allgemeinen Formel I, in der R eine Amino- oder Hydroxygruppe bedeutet, erfolgt in an sich bekannter Weise durch Umsetzung von 5-Brom-pyrimidionen der allgemeinen Formel II

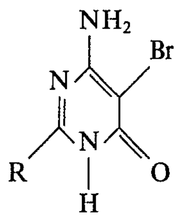

(II)

in der R die oben angegebene Bedeutung hat, mit 2-Aminothiophenol in Gegenwart von Basen, z. B. Kaliumcarbonat, in einem organischen Lösungsmittel bei Temperaturen zwischen 150 und 200°C.

Die als Ausgangsverbindungen eingesetzten Brompyrimidinone der allgemeinen Formel II sind in der Literatur beschriebene Verbindungen.

Die Verbindungen der allgemeinen Formel I können entweder als Basen oder in der Form ihrer Salze aus den Reaktionsgemischen isoliert werden.

Sie lassen sich als Basen mit geeigneten anorganischen oder organischen Säuren nach bekannten Verfahren in Salze überführen. Bevorzugt werden physiologisch verträgliche Salze. Hierfür sind als anorganische Säuren beispielsweise Halogenwasserstoffsäuren, zum Beispiel Salzsäure oder Schwefelsäure und als organische Säuren zum Beispiel Fumarsäure und Maleinsäure geeignet. Zur Herstellung wird die heiße alkalische Lösung der Base mit der alkoholischen Lösung einer geeigneten Säure versetzt und man erhält nach Ätherzusatz das Salz.

Die folgenden Beispiele dienen zur Erläuterung der Erfindung:

## Beispiel 1

### 6-Amino-5-(2-amino-phenylthio)-2,4(1 H, 3 H)-pyrimidindion (R=OH)

10,3 g 6-Amino-5-brom-2,4(1 H, 3 H)-pyrimidindion, 7,0 g Kaliumcarbonat und 9,0 g 2-Amino-thiophenol werden in 150 ml Ethylenglykol unter Stickstoffatmosphäre 4 Stunden auf 170°C erhitzt. Nach Filtration gießt man das Reaktionsgemisch auf 400 ml Wasser und dekantiert vom öligen Rückstand. Durch Ansäuern mit Essigsäure erhält man 4 g 6-Amino-5-(2-amino-phenylthio)-2,4(1 H, 3 H)-pyrimidindion, das durch Umkristallisation aus Ethanol/Wasser gereinigt wird.

Fp. >310°C.
IR-Spektrum (KBr): 3450, 3360, 3200 $cm^{-1}$ ($NH_2$, NH) 1710, 1620 $cm^{-1}$ (C=O, C=N—)

Das Hydrochlorid wird auf folgende Weise dargestellt: Man löst die Base in Methanol, gibt bis zur sauren Reaktion äthanolische Salzsäure zu und filtriert das in Methanol schwerlösliche 6-Amino-5-(2-amino-phenylthio)-2,4(1 H, 3 H)-pyrimidindion-Hydrochlorid ab. Fp. >300°C.

## Beispiel 2

### 2,6-Diamino-5-(2-amino-phenylthio)-4(1 H)-pyrimidinon (R=$NH_2$)

Analog Beispiel 1 wurde ausgehend von 2,6-Diamino-5-brom-4(1 H)-pyrimidinon das 2,6-Diamino-5-(2-amino-phenylthio)-4(1 H)-pyrimidinon hergestellt.

Fp. 294—295°C (Z.)
IR-Spektrum (KBr): 3430, 3370, 3150 $cm^{-1}$ ($NH_2$, NH), 1620 $cm^{-1}$ (C=O; C=N—)

Beispiel 3

Herstellung von Tabletten

Tabletten, welche die nachstehend angegebenen Bestandteile enthalten, werden nach bekannten Arbeitsweisen hergestellt. Diese sind für die Behandlung von Hypertonie und Ödemen in einer Dosierungsmenge von 250 mg zweimal täglich und für die Behandlung von Hyperuricämie mit einer Menge von 250 mg einmal täglich geeignet.

| | |
|---|---|
| 6-Amino-5-(2-amino-phenylthio)-2,4(1 H, 3 H)-pyrimidindion | 250 mg |
| Lactose | 448 mg |
| Maisstärke | 50 mg |
| Magnesiumstearat | 2 mg |

**Patentansprüche**

1. Substituierte 5-Phenylthio-6-amino-pyrimidinone der allgemeinen Formel I

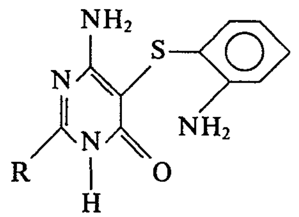

(I)

in der R eine Amino- oder Hydroxygruppe bedeutet, sowie ihre tautomeren Formen und deren Säureadditionssalze, ausgenommen 2,4-Diamino-5-(2-aminophenylthio)-6-hydroxypyrimidin.

2. 6-Amino-5-(2-amino-phenylthio)-2,4(1 H, 3 H)-pyrimidindion.

3. 2,6-Diamino-5-(2-amino-phenylthio)-4-(1 H)-pyrimidinon.

4. Verfahren zur Herstellung der 5-Phenylthio-6-aminopyrimidinone der allgemeinen Formel I, in der R eine Amino- oder Hydroxygruppe bedeutet, dadurch gekennzeichnet, daß man in an sich bekannter Weise eine Verbindung der allgemeinen Formel II

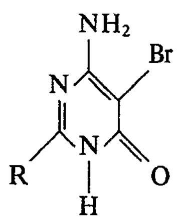

(II)

in der R die oben angegebene Bedeutung hat, mit 2-Amino-thiophenol umsetzt.

5. Pharmazeutisches Präparat, dadurch gekennzeichnet, daß es eine oder mehrere der Verbindungen gemäß der allgemeinen Formel I, in der R eine Amino- oder Hydroxygruppe bedeutet, oder deren physiologisch verträgliche Salze und gegebenenfalls übliche Trägerstoffe und/oder Verdünnungsmittel enthält.

6. Pharmazeutisches Präparat, dadurch gekennzeichnet, daß es 6-Amino-5-(2-amino-phenylthio)-2,4(1 H, 3 H)-pyrimidindion und seine tautomeren Formen oder deren physiologisch verträgliche Salze und gegebenenfalls übliche Trägerstoffe und/oder Verdünnungsmittel enthält.

7. Substituierte 5-Phenylthio-6-amino-pyrimidinone der allgemeinen Formel I

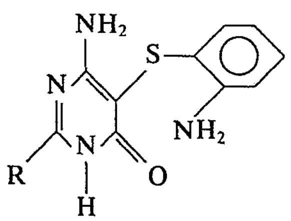

(I)

in der R eine Amino- oder Hydroxygruppe bedeutet, sowie ihre tautomeren Formen und deren Säureadditionssalze zur Behandlung von Hypertonie, Hyperuricämie und Ödemen.

**Claims**

1. Substituted 5-phenylthio-6-amino-pyrimidinones of the general formula I

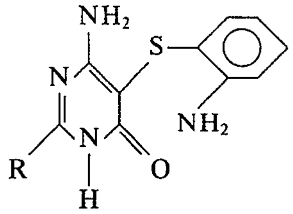

(I)

in which R denotes an amino or hydroxyl group, as well as their tautomeric forms and their acid addition salts, with the exception of 2,4-diamino-5-(2-aminophenylthio)-6-hydroxypyrimidine.

2. 6-Amino-5-(2-amino-phenylthio)-2,4(1 H, 3 H)-pyrimidine-dione.

3. 2,6-Diamino-5-(2-amino-phenylthio)-4-(1 H)-pyrimidinone.

4. Process for the preparation of the 5-phenylthio-6-amino-pyrimidinones of the general formula I, in which R denotes an amino or hydroxyl group, characterised in that, in a manner known per se, a compound of the general formula II

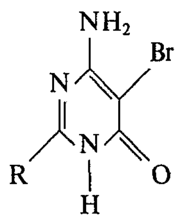

(II)

in which R has the abovementioned meaning, is reacted with 2-amino-thiophenol.

5. Pharmaceutical preparation, characterised in that it contains one or more of the compounds according to the general formula I, in which R denotes an amino or hydroxyl group, or their physiologically tolerated salts and, if appropriate, customary carries and/or diluents.

6. Pharmaceutical preparation, characterised in that it contains 6-amino-5-(2-amino-phenylthio)-2,4-(1 H, 3 H)-pyrimidine-dione and its tautomeric forms or their physiologically tolerated salts and, if appropriate, customary carriers and/or diluents.

7. Substituted 5-phenylthio-6-amino-pyrimidinones of the general formula I

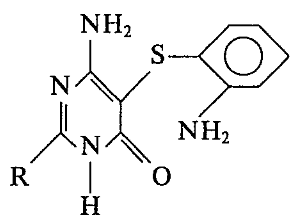

(I)

in which R denotes an amino or hydroxyl group, as well as their tautomeric forms and their acid addition salts, for the treatment of hypertonia, hyperuricaemia and oedemas.

**Revendications**

1. — 5-Phénylthio-6-aminopyrimidinones substituées de la formule générale I:

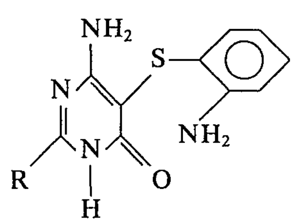

(I)

où R représente un radical amino ou hydroxyle, de même que leurs formes tautomères et leurs sels d'addition d'acides, à l'exception de la 2,4-diamino-5-(2-aminophénylthio)-6-hydroxypyrimidine.

2. — La 6-amino-5-(2-aminophénylthio)-2,4(1 H, 3 H)-pyrimidinedione.

3. — La 2,6-diamino-5-(2-aminophénylthio)-4-(1 H)-pyrimidinone.

4. — Procédé de préparation des 5-phénylthio-6-aminopyrimidinones de la formule générale I, où R représente un radical amino ou hydroxyle, caractérisé en ce qu'on fait réagir de manière connue un composé de la formule générale II:

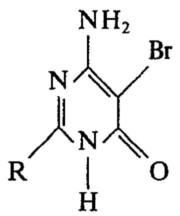

(II)

où R a la signification indiquée ci-dessus, avec le 2-aminothiophénol.

5. — Préparation pharmaceutique, caractérisée en ce qu'elle contient un ou plusieurs des composés de la formule générale I, où R représente un radical amino ou hydroxyle, ou leurs sels physiologiquement acceptables et éventuellement des excipients et/ou diluants habituels.

6. — Préparation pharmaceutique, caractérisée en ce qu'elle contient de la 6-amino-5-(2-aminophénylthio)-2,4(1 H, 3 H)-pyrimidinedione et ses formes tautomères ou leurs sels physiologiquement acceptables et éventuellement des excipients et/ou diluants habituels.

7. — 5-Phénylthio-6-aminopyrimidinones substituées de la formule générale I:

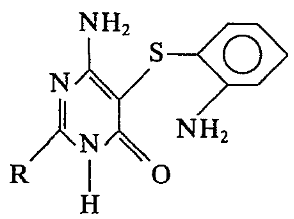

(I)

où R représente un radical amino ou hydroxyle, ainsi que leurs formes tautomères et leurs sels d'addition d'acides pour le traitement de l'hypertension, de l'hyperuricémie et de l'oedème.